# EUROPEAN PATENT APPLICATION

(11) **EP 1 693 381 A1**
(43) Date of publication of application: **23.08.2006**
(21) Application number: 04802449.1
(22) Date of filing: 10.12.2004
(51) Int. Cl.: C07K 14/47, C12N 15/12, C12N 15/63, C12N 15/10, A61K 38/17, A61P 31/04

(54) **GLYCINE-RICH PROTEINS, THEIR CODING SEQUENCES AND APPLICATIONS**

(30) Priority: 11.12.2003 CN 200310117354
(71) Applicant: Beijing Institute of Radiation Medicine, Beijing 100850 (CN)
(72) Inventor: ZHAO, Shifu, Beijing 100850 (CN); ZHANG, Yong, Beijing 100850 (CN); YIN, Lili, Beijing 100850 (CN)
(74) Representative: Goddar, Heinz J.
(86) International application number: PCT/CN2004/001435
(87) International publication number: WO 2005/056591

(57) **Abstract**

This invention relates to Glycine-rich protein (Glyrichin), as well as its coding gene and application, especially relates to the application for antibacterial purpose. The Glyrichin of the present invention are at least one selected from the following protein families: proteins having amino acid residues in Sequence 1, 3-14 in the Sequence List or protein with antibacterial activities having 1 to 20 amino acid residues of sequence 1, 3-14 being deleted, inserted and/or substituted and with 1 to 20 amino acid residue sequences being added to the carboxyl terminal or amino terminal of Sequence 1 and 3-14 of the Sequence List correspondingly. The Glyrichin of the present invention and the coding gene can be applied to antibacterial purpose, such as the preparation of drugs for prevention and/or treatment of human or livestock infectious bacterial diseases; for the preparation of products for prevention and/or treatment of potentially infectious bacterial diseases of different kinds of creatures; for the production of transgenic creatures which can defend against diseases and pests; for the preparation of the derivatives, antagonists as well as its ligands and antibodies of Glyrichin.

Sequence Listing

<110> BEIJING INSTITUTE OF RADIATION MEDICINE

Shifu, ZHAO

Yong, ZHANG

Lili, Yin

<120> Glycine-Rich Proteins, Their Coding Genes and Applications

<130> CPCNL60772

<150> CN200310117354.6

<151> 2003-12-11

<160> 14

<210> 1

<211> 79

<212> PRT

<213> Homo sapiens

<400> 1

Met Pro Val Ala Val Gly Pro Tyr Gly Gln Ser Gln Pro Ser Cys Phe
1               5                   10                  15
Asp Arg Val Lys Met Gly Phe Val Met Gly Cys Ala Val Gly Met Ala
            20                  25                  30
Ala Gly Ala Leu Phe Gly Thr Phe Ser Cys Leu Arg Ile Gly Met Arg
        35                  40                  45
Gly Arg Glu Leu Met Gly Gly Ile Gly Lys Thr Met Met Gln Ser Gly
    50                  55                  60
Gly Thr Phe Gly Thr Phe Met Ala Ile Gly Met Gly Ile Arg Cys
65                  70                  75

<210> 2
<211> 240
<212> DNA
<213> Homo sapiens

<400> 2
atgccggtgg ccgtgggtcc ctacggacag tcccagccaa gctgcttcga ccgtgtcaaa 60
atgggcttcg tgatgggttg cgccgtgggc atggcggccg gggcgctctt cggcaccttt 120
tcctgtctca ggatcggaat gcggggtcga gagctgatgg gcggcattgg gaaaaccatg 180
atgcagagtg gcggcacctt tggcacattc atggccattg ggatgggcat ccgatgctaa 240

<210> 3

<211> 80

<212> PRT

<213> Danio rerio

<400> 3

Met Pro Val Ser Val Gly Ser Tyr Gly Gln Gln Ala Gln Pro Ser Cys

1               5                   10                  15

Phe Asp Arg Val Lys Met Gly Phe Met Met Gly Phe Ala Val Gly Met

            20                  25                  30

Ala Ala Gly Ala Met Phe Gly Thr Phe Ser Cys Leu Arg Ile Gly Met

        35                  40                  45

Arg Gly Arg Glu Leu Met Gly Gly Val Gly Lys Thr Met Met Gln Ser

    50                  55                  60

Gly Gly Thr Phe Gly Thr Phe Met Ala Ile Gly Met Gly Ile Arg Cys

65                  70                  75                  80

<210> 4

<211> 128

<212> PRT

<213> Anopheles gambiae

<400> 4

Tyr Tyr Tyr Val Ile Val Val His Cys Cys Asp Asn Thr His Phe Asn

1               5                   10                  15

Glu Phe Val Pro Lys Ile Lys Leu Pro Pro Arg Lys Arg Tyr Val Arg

            20                  25                  30

Ser Gly Ser Phe Gln Ile Leu Gln Lys Thr Asp Thr Lys Ser Thr Met

        35                  40                  45

Pro Ala Val Pro Gly Gly Val Tyr Ser Gln Asn Gln Gln Pro Ser Cys

    50                  55                  60

Phe Asp Arg Met Lys Met Gly Phe Thr Ile Gly Phe Cys Val Gly Met

65                  70                  75                  80

Ala Ser Gly Ala Leu Phe Gly Gly Phe Ser Ala Leu Arg Tyr Gly Leu

                85                  90                  95

Arg Gly Arg Glu Leu Ile Asn Asn Val Gly Lys Val Met Val Gln Gly

            100                 105                 110

Gly Gly Thr Phe Gly Thr Phe Met Ala Ile Gly Thr Gly Ile Arg Cys

        115                 120                 125

<210>  5

<211>  79

<212>  PRT

<213>  Drosophila melanogas

<400>  5

Met Pro Leu Pro Thr Ser Ser Phe Ser Gln Gln Gly Pro Thr Cys Phe

1               5                   10                  15

Asp Lys Met Lys Thr Gly Phe Ile Ile Gly Phe Cys Val Gly Met Ala

            20                  25                  30

Ser Gly Ala Val Phe Gly Gly Phe Ser Ala Leu Arg Tyr Gly Leu Arg

        35                  40                  45

Gly Arg Glu Leu Ile Asn Asn Val Gly Lys Thr Met Val Gln Gly Gly

    50                  55                  60

Gly Thr Phe Gly Thr Phe Met Ala Ile Gly Thr Gly Ile Arg Cys

65                  70                  75

<210> 6

<211> 145

<212> PRT

<213> Caenorhabditis elegans

<400> 6

Met Pro Val Pro Ser Gly Tyr Ala Ala His Pro Gln Gly Ser Gln Pro

1               5                   10                  15

Ser Cys Phe Thr Lys Ile Arg Met Gly Leu Met Met Gly Ala Met Ile

            20                  25                  30

Gly Gly Ala Thr Gly Ile Leu Leu Gly Gly Phe Met Gly Phe Arg Ala

        35                  40                  45

Gly Met Arg Gly Lys Asp Leu Leu Leu Gln Thr Gly Lys Thr Val Ala

    50                  55                  60

Gln Ser Gly Gly Ser Phe Gly Val Phe Met Gly Val Ala Gln Gly Leu

65                  70                  75                  80

Arg Tyr Ile Phe Phe Lys Asn Leu Ala Gly Thr Gly Phe Trp Pro Phe

                85                  90                  95

Ser Leu Asn Phe Ser Arg Ser Ile Asp Asn Cys Pro Arg Gly Lys Val

            100                 105                 110

Val Tyr Ser Thr Arg Thr Asn Ala Phe Arg Phe Thr Thr Glu Ile Glu

        115                 120                 125

Lys Lys Glu Pro Arg Arg Asp Thr Gln Arg Ala Val Asn Leu Pro Gln

    130                 135                 140

Ile

145

<210> 7

<211> 162

<212> PRT

<213> Caenorhabditis elegans

<400> 7

Met Gln His Thr His Lys Glu Ala Asn Arg Arg Val Leu Gln Arg Lys

1               5                   10                  15

Lys Ile Asn Leu Leu Glu Met Ser Asp Lys Ile Cys Arg Asn Leu Ile

            20                  25                  30

Tyr Phe Gln Asn Phe Gln Ile Arg Met Gly Leu Met Met Gly Ala Met

        35                  40                  45

Ile Gly Gly Ala Thr Gly Ile Leu Leu Gly Gly Phe Met Gly Phe Arg

    50                  55                  60

Ala Gly Met Arg Gly Lys Asp Leu Leu Leu Gln Thr Gly Lys Thr Val

65                  70                  75                  80

Ala Gln Ser Gly Gly Ser Phe Gly Val Phe Met Gly Val Ala Gln Gly

                85                  90                  95

Leu Arg Tyr Ile Phe Phe Lys Asn Leu Ala Gly Thr Gly Phe Trp Pro

            100                 105                 110

Phe Ser Leu Asn Phe Ser Arg Ser Ile Asp Asn Cys Pro Arg Gly Lys

        115                 120                 125

Val Val Tyr Ser Thr Arg Thr Asn Ala Phe Arg Phe Thr Thr Glu Ile

    130                 135                 140

Glu Lys Lys Glu Pro Arg Arg Asp Thr Gln Arg Ala Val Asn Leu Pro

145                 150                 155                 160

Gln Ile

<210> 8

<211> 120

<212> PRT

<213>  Schizosaccharomyces pombe

<400>  8

Met Gln Ser Met Gln Pro Ser Thr Val Asp Lys Leu Lys Met Gly Ala

1               5                   10                  15

Ile Met Gly Ser Ala Ala Gly Leu Gly Ile Gly Phe Leu Phe Gly Gly

            20                  25                  30

Val Ala Val Leu Arg Tyr Gly Pro Gly Pro Arg Gly Phe Leu Arg Thr

        35                  40                  45

Leu Gly Gln Tyr Met Leu Thr Ser Ala Ala Thr Phe Gly Phe Phe Met

    50                  55                  60

Ser Ile Gly Ser Val Ile Arg Asn Glu Asp Ile Pro Leu Ile Gln Gln

65                  70                  75                  80

Ser Gly Ser His Trp Asn Gln Arg Leu Leu Asn Glu Asn Ala Asn Ser

                85                  90                  95

Ser Arg Ile Phe Ala Leu Ala Met Gln Gln Ala Lys Ser Ser Pro Arg

            100                 105                 110

Lys Ser Asn Glu Val Ala Glu Cys

        115                 120

<210>  9

<211>  113

<212>  PRT

<213>  Sacchromyces cerevisiae

<400>  9

<210>  10

<211>  74

<212>  PRT

<213>  Arabidopsis thaliana

<400> 10

Met Ala Lys Asn Ser Cys Leu Ala Lys Ile Thr Ala Gly Val Ala Val

1               5                   10                  15

Gly Gly Ala Leu Gly Gly Ala Val Gly Ala Val Tyr Gly Thr Tyr Glu

            20                  25                  30

Ala Ile Arg Val Lys Val Pro Gly Leu His Lys Val Arg Phe Ile Gly

        35                  40                  45

Gln Thr Thr Leu Ser Ser Ala Ala Ile Phe Gly Leu Phe Leu Gly Ala

    50                  55                  60

Gly Ser Leu Ile His Cys Gly Lys Gly Tyr

65                  70

<210> 11

<211> 168

<212> PRT

<213> Plasmodium falciparum 3D7

<400> 11

Met Met Asn Trp Phe Arg Lys Lys Asp Ser Asn Glu Asn Lys Lys Val

1               5                   10                  15

Lys Ser Glu Tyr Asp Glu Tyr Val Thr Pro Pro Pro Phe Gly Asn Tyr

            20                  25                  30

Leu Val Ser Glu Pro Lys Lys Pro Lys Ser Leu Lys Asn Asp Lys Thr

        35                  40                  45

Ala Ile Thr Glu Phe Lys Gly Phe Thr Pro Pro Pro Lys Phe Glu Phe

    50                  55                  60

Lys Glu Asp Ile Ser Asp Asn Lys Tyr Glu Glu Asp Phe Ser Lys Tyr

65                  70                  75                  80

Thr Ser Asn Asn Ile Ile Asp Ser Ser Phe Tyr Asp Asp Lys Lys Lys

                85                  90                  95

Leu Ser Asp Val Asn Leu Ser His Arg Thr Arg Ala Cys Phe Glu Ser

            100                 105                 110

Ile Lys Met Gly Val Lys Met Gly Thr Met Val Gly Gly Ile Phe Gly

        115                 120                 125

Ser Leu Thr Gly Ile Tyr Ala Ser Phe Ala His Lys Asn Leu Phe Ile

    130                 135                 140

Leu Pro Val Ser Val Leu Gly Gly Ala Val Ser Phe Gly Phe Phe Leu

145                 150                 155                 160

Gly Cys Gly Met Ile Val Arg Cys

                165

<210> 12

<211> 167

<212> PRT

<213> Plasmodium yoelii yoelii

<400> 12

Met Met Asn Trp Phe Lys Lys Lys Glu Thr Thr Glu Glu Pro Gln Val

1               5                   10                  15

Lys Ser Glu Tyr Asp Ser Tyr Val Thr Pro Pro Pro Phe Gly Asn Tyr

            20                  25                  30

Leu Ala Lys Lys Pro Glu Lys Pro Lys Ser Leu Lys Asn Glu Lys Ile

        35                  40                  45

Asn Val Thr Glu Phe Lys Gly Phe Thr Pro Pro Pro Lys Phe Glu Phe

    50                  55                  60

Lys Glu Asp Thr Thr Asp Thr Gln Tyr Asp Gln Asp Phe Ser Lys Tyr

65                  70                  75                  80

Thr Asn Asn Asn Phe Ile Asp Ser Ser Phe Tyr Asp Asp Lys Pro Asn

                85                  90                  95

Met Phe Asp Phe Thr Leu Ser His Arg Thr Lys Ala Cys Leu Glu Ser

            100                 105                 110

Val Lys Met Gly Val Lys Met Gly Thr Met Val Gly Gly Ile Phe Gly

        115                 120                 125

Ser Leu Thr Gly Leu Tyr Ala Ser Phe Ala His Lys Asn Leu Phe Ile

    130                 135                 140

Phe Pro Val Ser Val Ile Gly Gly Ala Val Ser Phe Gly Phe Phe Leu

145                 150                 155                 160

Gly Cys Gly Met Ile Val Arg

                165

<210> 13

<211> 107

<212> PRT

<213> Magnaporthe grisea

<400> 13

Met Pro Pro Pro Pro Arg Gln Ala Gly His Gly Ala Ala Pro Ser Asn

1               5                   10                  15

Met Asp Lys Pro Val Gly Ala Ile Met Gly Phe Ile Tyr Gly Thr Val

            20                  25                  30

Thr Val Phe Arg Gly Gly Ala Gly Pro Asn Gly Ile Met Arg Thr Ile

        35                  40                  45

Gly Gln Tyr Met Leu Ala Ser Gly Thr Thr Phe Gly Phe Phe Met Gly

    50                  55                  60

Ile Gly Ser Val Ile Arg Ser Asp Ala Ser Pro Ile Ser Gln Gln Ala

65                  70                  75                  80

Tyr Phe Gln Thr Arg Pro Arg Pro Leu Ile Met Ala Ser His Arg Ala

                85                  90                  95

Phe Arg Pro Gln Gln Ser Thr Arg Arg Asn Asp

            100                 105

<210> 14

<211> 129

<212> PRT

<213> Neurospora crassa

<400> 14

Met Pro Pro Pro Pro Gln His Gly Gly Ala Val Gly Pro Ser Asn Phe

1               5                   10                  15

Asp Lys Phe Lys Met Gly Ala Met Met Gly Gly Ser Pro Ser Pro Lys

            20                  25                  30

Asp Ser Ile Asp Leu Arg Ala Cys Pro Asp Val Arg His Ser Pro Gly

        35                  40                  45

Thr Val Asn Ile Phe Arg Tyr Gly Ala Gly Pro Asn Gly Ile Met Arg

    50                  55                  60

Thr Leu Gly Gln Tyr Met Leu Gly Ser Gly Ala Thr Phe Gly Arg Pro

65                  70                  75                  80

Ser Ser Phe Phe Met Ser Ile Gly Ser Val Ile Arg Ser Asp Ser Ser

                85                  90                  95

Pro Ile Val Ala Glu Ala Tyr Tyr Arg Ala Gln Arg Arg Pro Met Ile

            100                 105                 110

Met Ala Ala Gln Ala Phe Arg Pro Ala Tyr Tyr Pro Thr Arg Arg Ser

        115                 120                 125

Asp

## Description

### Technical Field

This invention relates to Glycine-rich protein (hereafter refer to Glyrichin), its coding gene and application, especially relates to Glyrichin of human and mouse origin and its coding gene, as well as its application in anti-bacterial area.

### Discussion of the Prior Art

Over the past two decades, the medical circle has not discovered any new families of antibiotics, on the other hand, drug resistance has developed very rapidly. It is troubling that after the problem of *Staphylococcus aureus* resistance has just been overcome, American doctors have found a new bacterial strain that is resistant to the latest antibiotic, Vancomycin. The problem of drug resistant pathogenic bacteria is increasingly threatening people's health. A completely novel type of antibiotic will be an effective way to resolve the resistant problem. As an active factor of organism's innate immunity, antimicrobial peptides are used by various organisms in defending against the invasion of pathogenic bacteria, which exist extensively in nature. The purified endogenous antimicrobial peptides have characteristics such as high antibacterial activity, broad antibacterial spectrum, many varieties, large scope for selection and low-degree of resistant mutation of target bacterial strains. Therefore, anti-microbial peptides are considered to have wide prospects for application in the medical industry, food industry and agriculture.

Antimicrobial peptide refers to the polypeptide synthesized on ribosome through encoded genes. Their relative molecular masses are typically less than 10kDa. Due to their antibiotic activities, they are also called polypeptide antibiotics. Most antimicrobial peptides are stable under high temperature and their activities can be retained even after being heated for 10 to 15min at 100°C. Antibacterial peptides have strong tolerance to higher ion strength and extreme pH values. Most of the antibacterial peptides have isoelectric points of >7 and show strong characteristics of positive ions. Meanwhile, some antimicrobial peptides have the capability to resist the hydrolysis by trypsases or pepsases. Additionally, studies have shown that antimicrobial peptides of different families have little homology in terms of sequence while different antibacterial peptides of the same family have high levels of homology in terms of sequence, which means that their function is highly conserved. In addition to their antibacterial and antifungal activities, some antimicrobial peptides also have antiprotozoal, antiviral, and anti-tumor activities. Antibacterial peptides exist widely in living nature. Up to now, more than 700 kinds of polypeptide antibiotics have been found in bacteria, fungi, amphibians, pests, higher plants, mammals and even humans. Currently, preclinical feasibility studies of many antimicrobial peptides are being carried out. Additionally, studies on transgenic animals and plants with antimicrobial peptides, the application of antimicrobial peptides in food preservative, preservation of flowers, cosmetics, seed coating and animal feed additives are also ongoing.

Given the promising prospects for application in the medical, food and agricultural industries, the search for novel natural antimicrobial peptides in nature through various ways has become a worldwide priority.

### Summary of the Invention

The objective of this invention is to provide a Glyrichin, its coding gene and their antibacteria effects.

Glyrichin provided by this invention is selected (cloned) from at least one of the following glytichin families:
1) Human Glyrichin (h Glyrichin) and mouse Glyrichin (m Glyrichin): protein having the amino acid sequence of the sequence 1 in the Sequence List or protein with antibacterial activities having sequence 1 in the Sequence List with 1 to 20 amino acid residues of it being deleted, inserted and / or substituted and with the 1 to 20 amino acid residues being added to the carboxyl terminal and / or amino terminal of sequence 1;
*2) Danio rerio* Glyrichin: protein having the amino acid sequence of the sequence 3 in the Sequence List or protein with antibacterial activities having sequence 3 in the Sequence List with 1 to 20 amino acid residues of it being deleted, inserted and / or substituted and with the 1 to 20 amino acid residues being added to the carboxyl terminal and / or amino terminal of sequence 3;
3) *Anopheles gambiae* Glyrichien: protein having the amino acid residue sequence of the sequence 4 in the Sequence List or protein with bacterial activities having sequence 4 in the Sequence List with 1 to 20 amino acid residues of it being deleted, inserted and / or substituted and with the 1 to 20 amino acid residues being added to the carboxyl terminal and / or amino terminal of sequence 4;
4) *Drosophila melanogas* Glyrichin: protein having the amino acid residue sequence of the sequence 5 in the Sequence List or protein with bacterial activities having sequence 5 in the Sequence List with 1 to 20 amino acid residues of it being deleted, inserted and / or substituted and with the 1 to 20 amino acid residues being added to the carboxyl terminal and / or amino terminal of sequence 5;
5) *Caenorhabditis elegans* Glyrichin: protein having the amino acid residue sequence of the sequence 6 in the Sequence List or protein with bacterial activities having sequence 6 in the Sequence List with 1 to 20 amino acid residues of it being deleted, inserted and / or substituted and with the 1 to 20 amino acid residues being added to the carboxyl terminal and / or amino terminal of sequence 6;
6) *Caenorhabditis elegans* Glyrichin: protein having the amino acid residue sequence of the sequence 7 in the Sequence List or protein with bacterial activities having sequence 7 in the Sequence List with 1 to 20 amino acid residues of it being deleted, inserted and / or substituted and with the 1 to 20 amino acid residues being added to the carboxyl terminal and / or amino terminal of sequence 7;
7) *Schizosaccharomyces pombe* Glyrichin: protein having the amino acid residue sequence of the sequence 8 in the Sequence List or protein with bacterial activities having sequence 8 in the Sequence List with 1 to 20 amino acid residues of it being deleted, inserted and / or substituted and with the 1 to 20 amino acid residues being added to the carboxyl terminal and / or amino terminal of sequence 8;
8) *Sacchromyces cerevisiae* Glyrichin: protein having the amino acid residue sequence of the sequence 9 in the Sequence List or protein with bacterial activities having sequence 9 in the Sequence List with 1 to 20 amino acid residues of it being deleted, inserted and / or substituted and with the 1 to 20 amino acid residues being added to the carboxyl terminal and / or amino terminal of sequence 9;
*9) Arabiopsis thaliana* Glyrichin: protein having the amino acid residue sequence of the sequence 10 in the Sequence List or protein with bacterial activities having sequence 10 in the Sequence List with 1 to 20 amino acid residues of it being deleted, inserted and / or substituted and with the 1 to 20 amino acid residues being added to the carboxyl terminal and / or amino terminal of sequence 10;
10) *Plasmodium falciparum* 3D7 Glyrichin: protein having the amino acid residue sequence of the sequence 11 in the Sequence List or protein with bacterial activities having sequence 11 in the Sequence List with 1 to 20 amino acid residues of it being deleted, inserted and / or substituted and with the 1 to 20 amino acid residues being added to the carboxyl terminal and / or amino terminal of sequence 11;
11) *Plasmodium yoelii yoelii* Glyrichin: protein having the amino acid residue sequence of the sequence 12 in the Sequence List or protein with bacterial activities having sequence 12 in the Sequence List with 1 to 20 amino acid residues of it being deleted, inserted and / or substituted and with the 1 to 20 amino acid residues being added to the carboxyl terminal and / or amino terminal of sequence 12;
12) *Magnaporthe grisea* Glyrichin: protein having the amino acid residue sequence of the sequence 13 in the Sequence List or protein with bacterial activities having sequence 13 in the Sequence List with 1 to 20 amino acid residues of it being deleted, inserted and / or substituted and with the 1 to 20 amino acid residues being added to the carboxyl terminal and / or amino terminal of sequence 13;
13) *Neurospora crassa* Glyrichin: protein having the amino acid residue sequence of the sequence 14 in the Sequence List or protein with bacterial activities having sequence 14 in the Sequence List with 1 to 20 amino acid residues of it being deleted, inserted and / or substituted and with the 1 to 20 amino acid residues being added to the carboxyl terminal and / or amino terminal of sequence 14;

The preferred glyrichins hereof are human glyrichin and mouse glyichin.

The number of amino acid residues that undergo deletion, insertion and/or substitution as well as addition at the carboxyl terminal and /or amino terminal is preferred to be 1 to 10, more preferred to be 1 to 5 and most preferred to be 1 to 3.

The preferred substitutions hereof are shown in Table 1.

**Table 1. List of substitutable amino acids of glyrichin family**

| Original residue | Representative Substitution | Preferred Substitution |
|---|---|---|
| Ala(A) | Val; Leu; Ile | Val |
| Arg(R) | Lys;Gln;Asn | Lys |
| Asn(N) | Gln;His;Lys;Arg | Gln |
| Asp(D) | Glu | Glu |
| Cys(C) | Ser | Ser |
| Gln(Q) | Asn | Asn |
| Glu(E) | Asp | Asp |
| Gly(G) | Pro; Ala | Ala |
| His(H) | Asn; Gln; Lys; Arg | Arg |
| Ile(I) | Leu; Val; Met; Ala ;Phe | Leu |
| Leu(L) | Ile; Val; Met; Ala; Phe | Ile |
| Lys(K) | Arg; Gln; Asn | Arg |
| Met(M) | Leu; Phe; Ile | Leu |
| Phe(F) | Leu;Val; Ile; Ala; Tyr | Leu |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala | Leu |

Glyrichin described in this invention can be modified (the Primary Structure is usually not affected), such as acetylation, carboxylation, glycosylation and phosphorylation.

The coding gene of the Glyrichin hereof also is within the scope of this invention.

The coding gene of the human Glyrichin (hGlyrichin) hereof may contain DNA sequence of the SEQID No: 2 in Sequence List or DNA sequence having 90% homology with the DNA sequence in the SEQID No: 2 of the Sequence List, a DNA sequence corresponding to the amino acid residue sequence in the coding Sequence List 1, or nucleotide sequences hybridized with DNA sequence in the sequence 2 of Sequence List under high strict conditions.

The high strict conditions for the hybridization refer to a wash of the blotting membrane in 0.1 × SSPE (or 0.1 ×SSC) and 0.1% SDS solution at 65°C.

The expression vectors, cell lines and engineered bacteria containing the coding gene of the Glyrichin hereof belong to the protection scope of this invention.

The Glyrichin in this invention hereof can be used for antibacterial purposes. Specifically, its application consists of the following aspects:
1) preparation of drugs for prophylaxis and/or treatment of human or livestock bacterial infectious diseases;
2) preparation of products for prevention and/or treatment of potentially infectious bacterial disease of various kinds of creatures;
3) production of transgenic organisms which can defend against diseases and pests;
4) preparation of derivatives, antagonists or ligands, or antibodies to Glyrichin.

### Brief Description of the Drawing

Fig. 1: The phylogenetic tree analysis of the Glyrichin family members in the comparison to the homology.
Fig. 2: Conserved structural domain of the Glyrichin family.
Fig. 3: Homology of the structural domain among different members of the Glyrichin family.
Fig. 4: Structural characteristics of the Glyrichin family.
Fig.5A: Northern blot analysis results of the size and number of hGlyrichin transcripts using hGlyrichin gene as the probe.
Fig. 5B: The results of the in vitro transcription and translation of hGlyrichin.
Fig. 6A: The semi-logarithm growth curve of E. coli BL21 transformed with pET-22b (+) plasmid with or without the induction of 0.5mM IPTG.
Fig. 6B: The semi-logarithm growth curve of E. coli BL21 transformed with pET-22b(+)-UBF and pET-22b(+)-PTH with or without the induction of 0.5mM IPTG.
Fig. 6C: The semi-logarithm growth curve of E. coli BL21 transformed with pET-22b-hGlyrichin positive clone 1 and 8 with or without the induction of 0.5mM IPTG.
Fig. 6D: The semi-logarithm growth curve of E. coli BL21 transformed with pET-22b-hGlyrichin positive clone 1 with or without the induction of 0.5mM IPTG.
Fig. 7: PAGE electrophoresis result of the prokaryocytic expression product of hGlyrichin.
Fig. 8: The inhibitory effects of the expression products of yeast transformed by full-length or 5'-deleted hGlyrichin gene on the growth of E. Coli BL21.
Fig. 9: The growth curves of transformed BL-21with pET-22b plasmids containing either fulllength, or 5'-deleted or 3'-deleted hGlyrichin gene with the induction of 0.5mM IPTG.

### Description of the Preferred Embodiment

The differentially expressed genes of mouse bone marrow stromal cells before and after LTC (long term culture) are screened through the Suppression Subtractive Hybridization (SSH) method and 131 differentially expressed EST clones were harvested. It is verified through bioinformatics analysis that these clones represent 26 genes which are functionally known or partially known and 7 completely novel genes. Among them, 5 have a complete open-reading frame including the mGlyrichin gene (GenBank number: AY028425) encoding amino acid sequence of mGlyrichin in Sequence 1.

After mGlyrichin was obtained, hGlyrichin with complete ORF sequence of 240bp is obtained through RT-PCR amplification using human embryonic liver mRNA as the template and primers designed based on the mGlyrichin sequence. The amino acid sequence of hGlyrichin is also obtained according to hGlyrichin nucleotide sequence. The sequence is then specifically analyzed with means of bioinformatics and the homology comparison (multiple-sequence alignment) was performed among the family members.

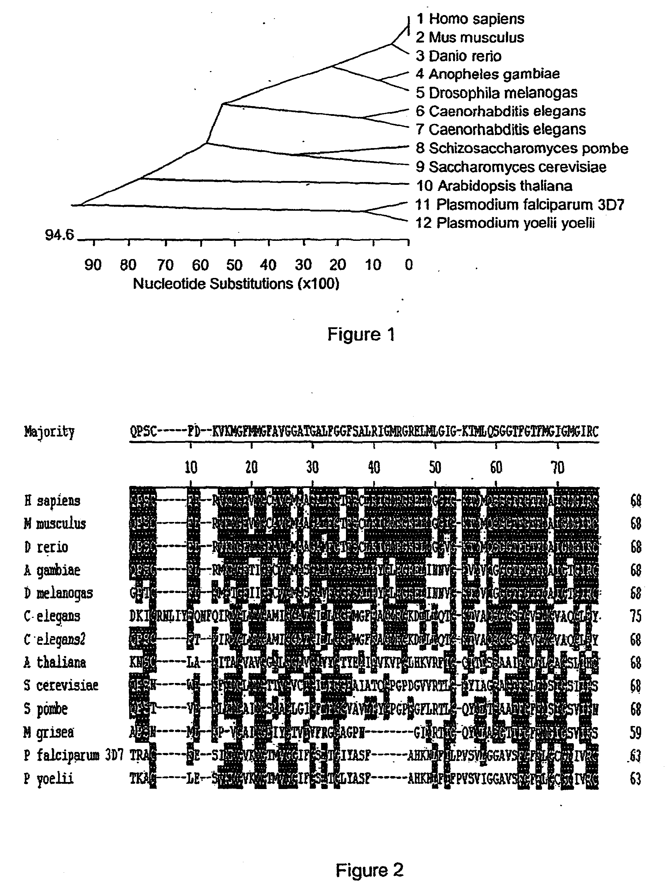

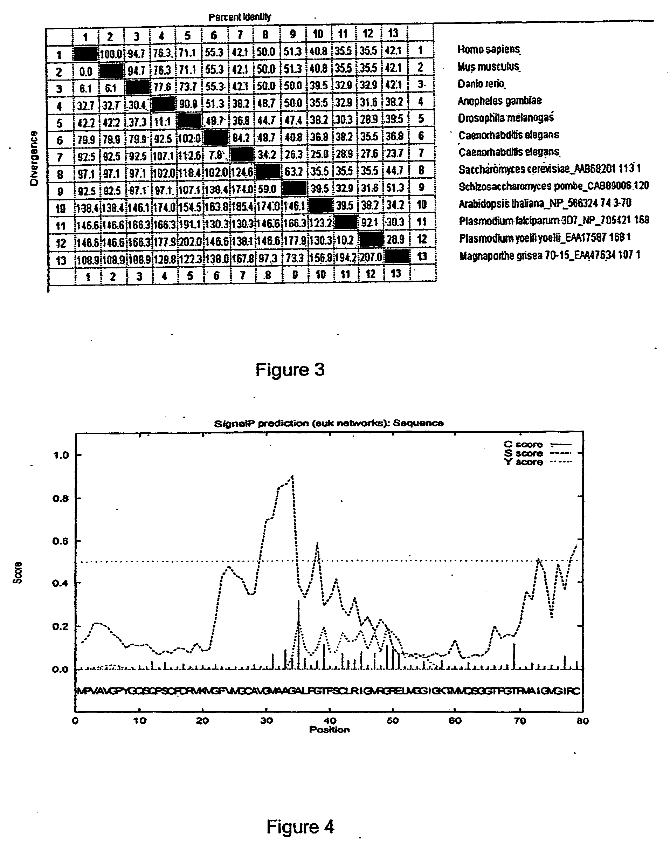

The phylogenetic tree is analyzed within 12 sequences in Figure 1 in order to study the evolutionary relationships among Glyrichin family members. The evolution of Glyrichin genes from lower to higher organisms and the genetic relationships among the family members are presented in Figure 1. The conserved Glyrich domains of Glyrichin family members (conserved amino acids are shown in Reverse) are presented in Figure 2. The sequence homology of Glyrich domains among different Glyrichin family members are presented in Figure 3, which demonstrates the close genetic relationships of every two members. Results of electronic PCR showed that the hGlyrichin gene is located in the 20q11.21 area of human chromosome and consists of 3 exons encoding a small protein of 79 amino acids. Homology comparison with BlastP of amino acid sequences shows that this gene is very conserved in evolution, and exists in various species including fungi (e.g. *Neurospora crassa* and yeast), plants (e.g. *Arabidopsis thaliana*), *Plasmodium falciparum* 3D7, *Caenorhabditis elegans, Drosophila melanogas, Anopheles gambiae, Danio rerio,* mice and human, and forms the homologous Glyrichin protein family. Homology comparison of the genes derived from various organisms shows that the highest homology, 100% homology, is demonstrated between hGlyrichin and mGlyrichin, 90% homology between hGlyrichin and *Danio rerio* Glyrichin, 62% homology between hGlyrichin and *Drosophila melanogas* Glyrichin, 46% homology between hGlyrichin and yeast Glyrichin. The genetic relationships between different family members are presented with a cladogram (Figure 1).

Among Glyrichin family members, the sequence characterizations are presented in Table 2.

**Table 2. Sequence characterization of Glyrichin family members with sequences of amino acid residues in Sequence 1 and Sequence 3 to 14**

| Species Name | Length of Polypeptide (aa) | Isoelectric Point | Number of Electric Charges (pH7. 0) | Glycine Content(%) |
|---|---|---|---|---|
| *Homo sapiens* | 79 | 9.36 | 4.79 | 21.52 |
| *Mus musculus* | 79 | 9.36 | 4.79 | 21.52 |
| *Danio rerio* | 80 | 9.81 | 4.82 | 21.2 |
| *Anopheles gambiae* | 128 | 9.73 | 10.08 | 14.1 |
| *Drosophila melanogaster* | 79 | 9.66 | 4.82 | 20.3 |
| *Caenorhabditis elegans* | 145 | 10.79 | 12.01 | 14.48 |
| *Schizosaccharomyces pom be* | 107 | 10.04 | 4.05 | 13.08 |
| *Saccharomyces cerevisiae* | 112 | 9.74 | 5.04 | 13.27 |
| *Plasmodium falciparum 3D7* | 168 | 9.66 | 5.15 | 15.94 |
| *Plasmodium Yoelii Yoelii* | 167 | 9.16 | 4.15 | 15.28 |
| *Magnaporthe grisea* | 107 | 11.75 | 8.24 | 13.08 |
| *Arabidopsis thaliana* | 74 | 9.84 | 6.18 | 14.86 |

Human Glyrichin (hGlyrichin) is composed of 79 amino acid residues, with a molecular weight of 8.8kDa and isoelectric point of 9.36. The net positive electrical charge is 4.79 at pH 7 and the glycine content is 21.52%. The secondary structure and the characterizations of hGlyrichin have been analyzed by the inventor. Figure 4 is the SignalP analysis result. The isoelectric points of the other members of Glyrichin family are all above 7 at pH 7 and they all have net positive electrical charge and strong hydrophobic domains. The above results show that the Glyrichin family has the typical structural features of known antimicrobial peptides.

The 13 members of Glyrichin family in this invention are from the species whose genome sequencing has been finished, covering from fungi to human. Based on this, a person skilled in the art can simply infer that they might exist in the species whose genome sequencing has not yet been finished. The common feature in the structure of Glyrichin family members is that they all contain an area rich in glycine that is between 59 to 68 amino acids in length. This area almost covers the whole sequences of hGlyrichin and mGlyrichin. Besides the common area rich in glycine, Glyrichin existing in other species also contains other sequences (such as signal peptide etc.) at the amino terminal or carboxyl terminal of this conserved area, but they are not conserved (Figure 2 and 3). This shows that the conserved area rich in glycine might represent basic biological activities of Glyrichin family members and that the redundant sequences were gradually eliminated in the process of biological evolution, making the size of molecule smaller and the structure more compact.

The coding genes of Glyrichin in this invention can be used to express or produce recombinant Glyrichin protein through routine recombinant DNA technology. The method of expression or production is as follows:
(1) Use the recombinant expression vectors containing the Glyrichin coding gene mentioned above to transform host cells;
(2) Cultivation of the host cell;
(3) Separate and purify protein from the medium or cells.

The starting vectors used to construct the recombinant expression vector can be the known vectors in this area, such as bacteria plasmid, bacteriophage, yeast plasmid, plant cell virus, mammal cellular virus including adenovirus or other vectors. In general, those plasmids and vectors that can replicate and express stably in host cells are applicable.

An important feature of expression vectors is that they usually contain a replication origin, promoter, marker gene and translational control component. The methods known to those skilled in the art can be used to construct expression vectors containing Glyrichin DNA coding sequence and appropriate transcription/ translation control signals.

Those skilled in the art know how to choose appropriate vectors, promoter, enhancer and type of host cells. The transformation of host cells with the above mentioned recombinant expression vectors may be conducted with the routine techniques known to those skilled in the art. The obtained transformants can be cultured with routine methods and Glyrichin protein can be expressed intracellular or on cell membrane, or can be excreted to the outside of the cells. If necessary, the recombinant protein can be separated and purified with various separation methods according to its physical, chemical and other characteristics. Other drugs, such as antibiotics including penicillin can be used in combination with Glyrichin protein in this invention.

In this invention, terms including "Glyrichin", "Glyrichin family member", "Glyrichin protein " or "Glyrichin polypeptide" are interchangeable, and all of them refer to protein or polypeptide with amino acid sequence (sequence 1 or sequence 3-14 in the Sequence List 1) of the family members of natural antimicrobial peptide Glyrichin. They include those family members of natural antimicrobial peptide Glyrichin with or without starting methionine as well as Glyrichin protein with or without signal peptide.

The term "Glyrichin structural domain" refers especially to a segment of the most conserved polypeptide in Sequence 1 to 13 in the Sequence List with 59 to 68 amino acid residues. Their isoeletric points are greater than 7.0 and they have net positive electrical charges at pH 7.0. The Glycine content in this conserved domain is the highest of the 20 amino acids and there is at least one hydrophobic region. Their sequences and structural features are presented in Figures 3 and 4 and Table 2.

The term "Glyrichin family" refers specifically to polypeptides that have a Glyrich domain and the homology of amino acids in this domain is 30% or higher, including the 13 polypeptides mentioned in this invention and other homologous polypeptides not reported. From the view of evolutionary biology, they are from the same origin.

Fusion proteins containing Glyrichin polypeptide or its segments are also involved in this invention. Besides polypeptides of almost full length, soluble segments of Glyrichin polypeptide are also included in this invention. Usually, the segments contain a certain number of consecutive amino acid sequences in the Glyrichin polypeptide sequence. It should be understood that the polypeptides in this invention are not limited to the representative polypeptides that are cited above.

The term "modification" (usually the primary structure is not changed) includes: in vivo or in vitro chemical modification of polypeptides such as acetylation or carboxylation. Modification also includes glycosylation, for example, the polypeptides formed in the process of synthesis and processing or further processing through glycosylation. The modification can also be accomplished through exposure of polypeptide to enzymes that catalyze glycosylation (e.g. glycosylase or deglycosylase of mammals). Modified forms also include the sequence containing phosphorylated amino acid residues (such as phosphoserine and phosphothreonine) as well as polypeptide whose proteolytic resistance or solubility is improved through modifications.

The function of the Glyrichin gene family has been representatively studied by the inventor using hGlyrichin and mGlyrichin as examples. Results show that hGlyrichin and mGlyrichin have antibacterial activities, which are extensively expressed in various tissues of humans and mice. The antibacterial peptides exist naturally and are likely to be helpful in the medical industry and all fields that require the prevention of bacterial infection. This is also the basic function and activity of Glyrichin family members.

This invention is further explained below using the cloning, expression, purification and antibacterial activity tests of hGlyrichin as an example. It should be understood that these examples should only be used to explain this invention instead of confine it. The experimental methods without stated conditions usually use standard conditions such as the conditions described in Molecular Cloning: Laboratory Brochure (New York: Cold Spring Harbor Laboratory Press 1989) by Sambrook *et al.* or the conditions recommended by manufactures.

### Example 1: Acquisition and analysis of hGlyrichin gene

### 1. Acquisition of the complete ORF sequence of hGlyrichin gene

The following pair of primers is designed based on the mGlyrichin gene and RT-PCR reaction is conducted using human fetal liver RNA as the template.
5'primer Pa: 5'-CGATGCCGGTGGCCGTGGGTCCCT-3'
3'primer Pb: 5'-TTAGCATCGTATGCCCATTCCA-3'

PCR reaction system: PCR reaction system is done according to standard molecular biology techniques. The amplification conditions of PCR are as follows: 4 min at 94°C for 1 cycle; 40 sec at 94°C, 50 sec at 60°C, 1 min at 72°C for 30cycles; 7 min at 72°C for 1 cycle. PCR products are purified using a Wizard PCR Purification kit (purchased from Promega), PCR product is sub-cloned into pGEM-T vector with T4 ligase to form pGEM-T/hGlyrichin, and then transformed into JM109 for sequencing. Results show that the Glyrichin gene has the nucleotide sequence of sequence 2 in the Sequence List and the coding sequence of amino acid residues in the coding Sequence List 1.

### 2. Northern blot analysis

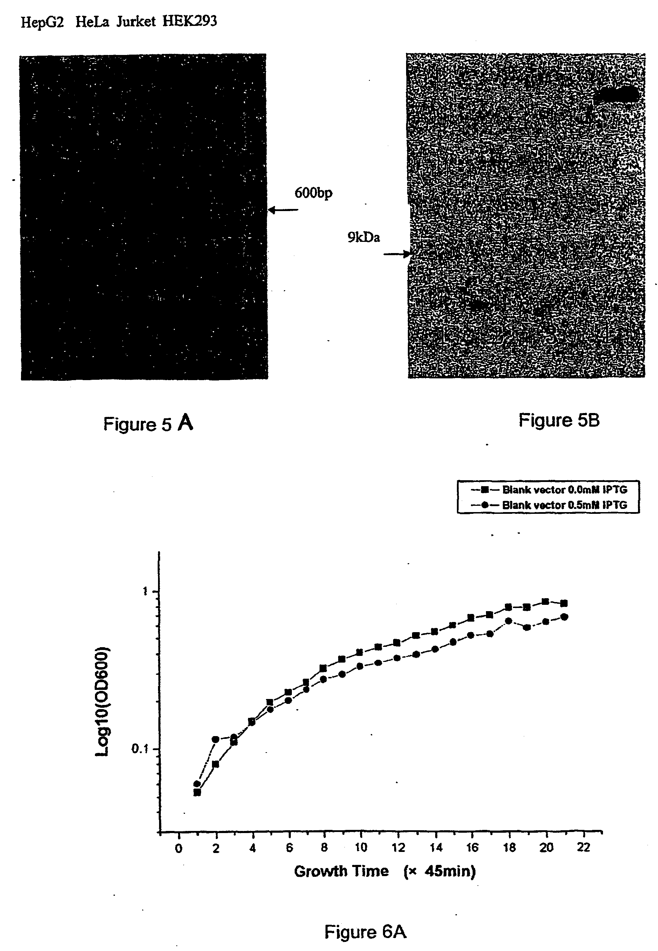

Four cell lines from human tumors, HepG2, HeLa, Jurket and HEK293, are cultured and the total RNAs are extracted, respectively, with a Wizard Plus mRNA purification kit (purchased from Promega). Twenty mg of each total RNA is sampled and separated on a 1.2% formaldehyde denaturing agarose gel, and then transferred onto a Hybond-N+ nylon membrane. It is then labeled with the a Prime-a-Gene kit from Promega using the complete ORF of hGlyrichin as the probe. The hybridization results are presented in Figure 5A, which shows that the hGlyrichin gene is expressed in the four tumor cell lines from different tissue sources, indicating that hGlyrichin may be an extensively expressed natural antibacterial peptide. The results also demonstrate that there is only one transcript with a size of approximately 600bp.

### 3. In vitro transcription and translation test

The full length ORF of hGlyrichin gene was obtained though PCR using pGEM-T/hGlyrichin plasmid as the template and the following primers:
5'primer: 5'-CGGGATCCCGATGCCGGTGGCCGTGGGTCCCT-3'
3'primer: 5'-GCTCGAGTTAGCATCGGATGCCCATCC-3'

The regular (refer to short protocols in molecular biology) PCR reaction system is adopted and the conditions of PCR amplification are as follows: 4 min at 94°C for 1 cycle; 40 sec at 94°C, 50 sec at 60°C, 1 min at 72°C for 30cycles; 7 min at 72°C for 1 cycle.

The amplification products of PCR are then incised with BamHI and SacI; pT7 vectors (purchased from Promega) are incised with the same enzymes and then linked with T4 DNA ligase to construct pT7-hGlyrichin plasmid. The constructed plasmids are then transcribed and translated in the following system:
- 25 µl system::
- Rabbit Reticulocyte lysate (RRL):: 12.5µl
- Reaction buffer:: 1.0 µl
- Amino acids mixture:: 0.5 µl
- ³⁵S-Met:: 1.0 µl(50 µli)
- DNA: 2.0 µl(0.5 µg/µl)
- RNase inhibitor:: 0.5 µl
- T7 DNA polymerase:: 0.5µl
- Deionized water:: 7.0µl
- Total volume:: 25.0 µl

The system is incubated at 30°C for 90 minutes. Five µl of sample is taken after the reaction is finished and then 10µl loading buffer is added into the sample to perform the SDS-PAGE. The gel is fixed in a fixing solution for 30 minutes, immersed in the desiccant for 5 minutes, and dried over night on a drying rack. Autoradiography is then performed and the film is exposed at -20°C for 24 hours. The results are presented in Figure 5B. The results demonstrate that the size of the protein encoded by the hGlyrichin gene is about 8.8 kDa after *in vitro* translation, which is the same as the results deduced by theory. This indicates that the gene can be transcribed and translated normally *in vitro.* The reagents used in the reaction system are from the TNT kit from Promega, and ³⁵S-Met is purchased from Amersham Bioscience Corp. In Figure 5B, Lane 1 is the molecular weight reference, Lane 2 is the UBF of unrelated genes (GenBankUBF-fl AF294842 ; Chinese Journal of Applied Physiology, 20:66, 2004), Lane 3 is the translation products of hGlyrichin gene and Lane 4 is the translation products of the positive control provided in the kit.

### Example 2: The inhibitory effects of hGlyrichin (mGlyrichin) on the growth of Escherichia coli BL21 transformed with different plasmid containing hGlyrichin gene or other genes with or without IPTG induction.

### 1. The construction of pET-22b-hGlyrichin

Use pGEM-T/hGlyrichin as the template, and 5'-GGAATTCCATATGCCGGTGGCCGTG GGTC-3' as well as 5' CCGCTCGAGTTAGCATCGGATCCCATC-3' as primers, amplify hGlyrichin gene through standard PCR amplification. The PCR reaction system (excluding primer) and reaction condition are the same as those of step 3 in Example 1.

Use NdeI and XhoI enzymes to cleave, then run a gel, purify the amplification products of hGlyrinchin, and sub-clone into expression vector pET-22b (+) that has been cleaved by NdeI and XhoI enzymes in the presence of T4 DNA ligase. Transform *E. coli* BL21 and then identify the positive clones 1 and 8 (containing plasmid pET-22b-hGlyrichin) by means of enzyme cleavage.

### 2. Construction of Control Plasmids pET-22b(+)-UBF and pET-22b(+)-PTH

Introduce the UBF gene (GenBankUBF-fl AF294842; Chinese Journal of Applied Physiology, 20:66, 2004) and the PTH gene (the 34 peptide gene sequence of parathyroid hormone) (GenBankNM000315) into the multiple cloning sites of pET-22b(+) vector respectively, thus obtain the control plasmids pET-22b(+)-UBF and pET-22b(+)-PTH.

### 3. Tests of growth inhibition

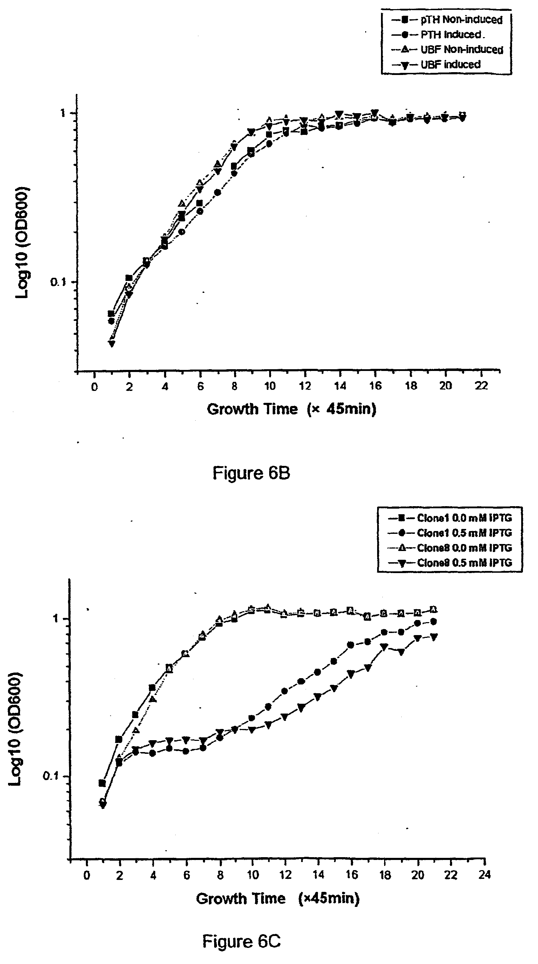

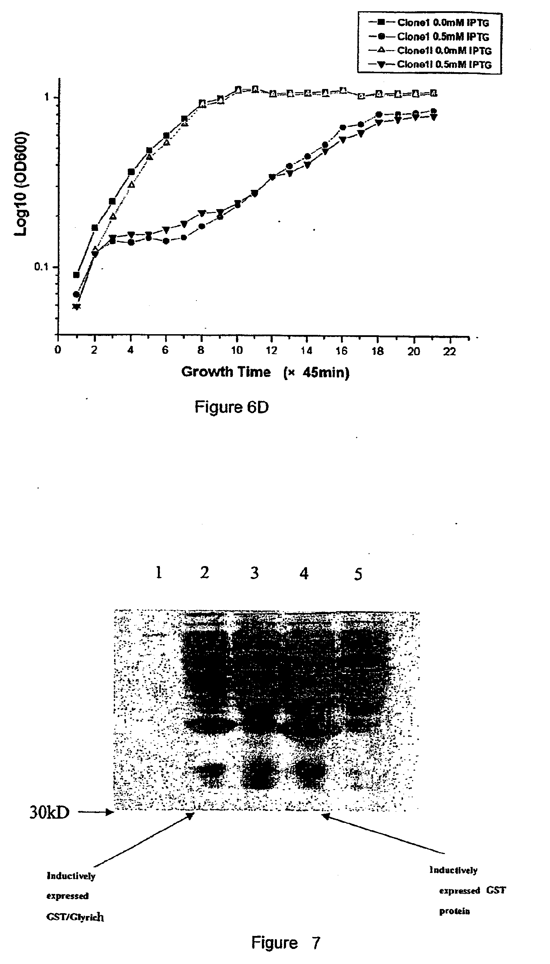

Select the positive clones and inoculate them into Liquid LB Culture Medium (containing 50µg/ml ampicillin). Incubate them for 12 hours in the shaker, 250rpm at 37°C. Reinoculate them into a test tube in 1:100 (v/v) ratio and continue to incubate them until the OD value is 0.03. Then add IPTG into each tube to a final concentration is 0.5mM and add the same volume of PBS to the negative control tube. Culture the positive and negative groups in a shaker, 250rpm, at 30°C; then take out 1ml of culture from each tube every 45 minutes and take an OD600 measurement in succession for ten times or more. Draw a Growth Curve with time as the horizontal axis and the logarithm of OD600 as the longitudinal axis. The results are shown in Figures 6A, 6B, 6C and 6D. Figure 6A showed that the bacterial growth curves basically coincide with each other whether IPTG is added to the bacterial strain transformed with pET-22b(+) blank Vector or IPTG is not added. Figure 6B shows that there is no influence on the growth of bacteria transformed with plasmids containing UBF or PTH gene regardless of whether IPTG is added or not. Figure 6C and 6D shows that the growth curves of the bacteria transformed with plasmid containing hGlyrichin are obviously different between IPTG induced or uninduced. The bacterial growth is obviously inhibited when the bacteria transformed with hGlyrichin-containing gene is induced by IPTG. From Figures 6C and 6D, it appears that as the time of culture prolongs, the IPTG is depleted and the bacterial growth resumes again. However, there is no such phenomena when the bacteria are transformed with unrelated gene-containing plsmids. These results fully show that the growth inhibitory effects are exerted by Glyrichin protein itself.

### Example 3: The purification of expression products of hGlyrichin and its inhibitory effects on the growth of bacteria

Primers designed are upstream 5'-CGGGATCCCGATGCCGGTGGCCGTGGTCCCT-3' and down stream 5'-GGAATTCTTAGCATCGTATGCCCATTCCA-3' in accordance with the cDNA sequence of mGlyrichin. PCR amplification is performed using human fetal liver mRNA after reverse transcription as the template. The PCR reaction system (except primer) and reaction conditions are the same as those of Step 1 in Example 1. After purification, the purified PCR products are sequenced and digested with restriction endonuclease BamHI and EcoRI, and then inserted into prokaryotic expression vector pGEX4-4T2 (construct from Pharmcia) that have been digested by the same restriction endonucleases in the presence of T4 DNA ligase. Transform E. coli JM109, induce expression *in vitro* for 5 hours with 0.5mM IPTG, and collect the bacteria. Resuspend the bacteria with PBS and carry-out several freeze-thaw cycles repeatedly. Centrifuge at 4°C, 12000rpm for 20 minutes and then take some of the supernatant for SDS-PAGE. The results are shown in Figure 7, which show that the fusion protein of GST-Glyrichin is expressed in the supernatant and its molecular weight is 34kD. Lane 1 Figure 7 is protein molecular weight standards, Lane 2 is the induced expression products of GST-hGlyrichin fusion protein, Lane 3 is the non-induced expression products of GST-hGlyrichin fusion protein, and Lane 4 is the induced expression products of GST protein, and Lane 5 is the non-induced expression products of GST protein.

Purify the GST-Glyrichin fusion protein obtained by induced expression with a Sepharose 4B GST purification column (Pharmcia Inc.), cleave with Enterokinase (Roche) to obtain free hGlyrichin protein. The antibacterial activities (against *E. coli* DH5α and *Bacillus subtilis* DB430) of GST-Glyrichin fusion protein and free hGlyrichin protein are assayed by means of a 96-well plate and minimal inhibition concentration (MIC) is determined. When the antibacterial activity is assayed, dilute the bacterial liquid to a concentration between 10⁴-10⁵CFU/ml, inoculate the bacterial liquid into the 96-well plate, 80µl per well. Dilute the polypeptides at a certain ratio and add 5µl per well. Cultivate the 96-well plate at 37°C for 12 hours, then measure the OD600 value with a UV/Vis spectrophotometer. The results show that the GST-Glyrichin fusion protein has no inhibitory effect on the growth of bacteria while free Glyrichin has inhibitory activity on the growth of bacteria at relatively low concentration. Free Glyrichin protein has antibacterial activity and can inhibit the growth of Gram+ bacteria and Gram- bacteria at relatively low concentrations (Table 3).

**Table 3. MIC of free hGlyrichin protein**

| | Name of bacterial strain | MIC Concentration (ug/ml) |
|---|---|---|
| G- Bacteria | *E. coli* DH5α | 0.2 |
| G+ Bacteria | *Bacilllus subtilis* DB430 | 3 |

### Example 4: The yeast expression of hGlyrichin and the assay of the antibacterial activity of the expression products

PCR primers are as follows: the full-length upstream primer: 5'-AGGAATTCATG CCGGTGGCCGTGGGTCCCTAC-3' ; 5'-deleted upstream primer: 5'-AGGAATTCATGGGCTT CGTGATGGGTTGC-3; full-length downstream primer: 5'-AAGGAAAAAAGCGGCCGCTTA GCATCGGATGC CCATCCCAATG-3'). PCR amplification is carried out respectively using pET-22b plasmid containing the full length of hGlyrichin as the template, in the presence of either a pair of upstream and downstream primers for full-length hGlyrichin or 5'-deleted upstream primer and full length downstream primer for 5'-deleted hGlyrichin. The 5'-deleted gene refers to a deletion of 60 bases at the 5'-terminal of hGlyrichin. The PCR reaction system (except primer) and reaction conditions are the same as those in step 3 of Example 1.

Insert the full-length and 5'-deleted fragment of the hGlyrichin gene obtained through PCR amplification, respectively, into pPIC9K Yeast Expression Plasmid (Invitrogen) between the EcoRI and NotI sites in the presence of T4 DNA ligase and transform *E. Coli* BL-21 engineering bacteria. Screen and obtain the constructed plasmids containing either full-length or 5'-deleted hGlyrichin. Linearize the highly purified plasmids with SalI endonuclease, perform electro-transforming method to transform GS115 yeast, and obtain positive clones by screening with a MD plate containing G418 (50µg/ml). Inoculate the positive clone into the culture media containing 5 ml BMGY and cultivate it in a shaker at 30°C until the OD600 is 2.0 to 6.0. Then dilute with BMMY culture media (containing methanol to a final concentration 1%) until the OD600 is 1.0 and continue culturing at 30°C. For the induction, add methanol every 24 hours until the final concentration is 0.5%, pipette 1ml of culture at different time intervals post induction into Eppendorff centrifuge tubes, centrifuge at 16000 rpm, and collect the supernatant for activity assay using the Kirby-Bauer disk diffusion test. The test is performed by applying the bacteria (*E. coli* BL-21) on to a LB plate containing 1% agar and then placing a filter paper of 2 mm diameter at the center of each small block. Drop 15µl of different cloning supernatants onto the filter paper, then drop the same volume after 20 minutes; drop three times in total. Culture the LB plate at 37°C for 4 more hours until the antibacterial rings can be observed, accordingly, the positive clones are determined. In this test, use 10µl ampicillin at the concentration of 100mg/ml as the positive control. After screening, positive clones expressing either full-length hGlyrichin (L4 clone) or 5'-deleted hGlyrichin (clone of S2, S12 and S5) are obtained. The inhibitory effects of L4, S2, S12 and S5 clones are shown in Figure 8, which shows that the supernatant of positive clones (L4, S2, S12 and S5) expressing either the full-length or 5'-deleted hGlyrichin gene markedly inhibit the growth of *E. coli* BL-21 on the surface of the agar plate. The experimental results described above verify that the yeast expressing system can express hGlychirin protein. Furthermore, its expression products, full-length or 5'-deleted, have antibacterial activities. These results lay a very solid basis for the future discovery of the definite structure responsible for the activity of this gene. According to the experimental results described above, persons skilled in the art can easily screen for the smallest hGlyrichin protein molecule with similar activity through similar methods.

### Example 5: Studies of the structure-activity relationships of hGlyrichin gene

Growth comparison of different transformants transformed with plasmids containing various deleted hGlyrichin gene

The inventor designed a series of primers according to the sequence of hGlyrichin:
Primer 1: 5'-GGAATTCCATATGCCGGTGGCCGTGGGTC-3'
Primer 2: 5'-GGAATTCCATATGGGCTTCGTGATGGGTTGC-3'
Primer 3: 5'-CCGGCTCGAGTTAGAATGTGCCAAAGGT-3'
Primer 4: 5'-CCGCTCGAGTTAGCATCGGATGCCCATC-3'

In order to obtain different length fragments of the hGlyrichin gene, different combinations of primers are used to create full length (primer 1 and 4 combination), 5'-deleted sequence (primer 2 and primer 4 combination), and 3'-deleted sequence (primer 1 and primer 3 combination) by PCR amplifications using pEt-22b-hGlyrichin as the template. The PCR reaction systems (primer excluded) and reaction conditions are the same as those in Step 3 of Example 1. A series of plasmids containing hGlyrichin gene fragments of different lengths are constructed by inserting each fragment deriving from PCR into pET-22b vector by using the method in Example 2. After transformation of *E. Coli* BL-21, screening of clones and identification, the transformed bacterial clones containing different constructed plasmids are used to induce the expression of different fragments of hGlyrichin using the method in Example 2 in order to observe the effect of the full length and different deleted fragments on the growth of transformed bacteria. The results are shown in Figure 9, which show that there are no inhibitory effects when *E. coli* BL-21 is transformed with vacant vector and expression induced with or without IPTG. When transformed with plasmid containing full-length or deleted fragment of hGlyrichin, the growth of bacteria is obviously inhibited within 5 hours after IPTG induction; however, the growth is not affected if IPTG is not added. In the Figure 9, the x-axis is the time (hr) (in the bracket) after IPTG induction and the y-axis is the value of OD600 absorbance. The above results demonstrate that some nucleotide sequences at the 5'-terminal and 3'-terminal of the hGlyrichin gene are not necessary for its antibacterial effect. The (-) in Figure 9 indicates that IPTG is not added, while (+) indicates that 0.5mM IPTG is added.

### Industrial Applicability

From the experimental results, the inventor for the first time discovers a Glyrichin family containing conserved sequences with antibacterial activity. Glyrichin can be applied in medical fields and all those fields where antibiotics are needed. Thus it has very broad application prospects.

## Claims

1. A Glyrichin selected from at least one of the following protein family:
1) Human Glyrichin (hGlyrichin) and mouse Glyrichin (mGlyrichin): protein having the amino acid residue sequence of the sequence 1 in the Sequence List or protein with antibacterial activities having sequence 1 in the Sequence List with 1 to 20 amino acid residues of it being deleted, inserted and / or substituted and with 1 to 20 amino acid residues being added to the carboxyl terminal and / or amino terminal of sequence 1;
2) Daniorerio Glyrichin: protein having the amino acid residue sequence of the sequence 3 in the Sequence List or protein with antibacterial activities having sequence 3 in the Sequence List with 1 to 20 amino acid residues of it being deleted, inserted and / or substituted and with 1 to 20 amino acid residues being added to the carboxyl terminal and / or amino terminal of sequence 3;
3) Anopheles gambiae Glyrichicn: protein having the amino acid residue sequence of the sequence 4 in the Sequence List or protein with antibacterial activities having sequence 4 in the Sequence List with 1 to 20 amino acid residues of it being deleted, inserted and / or substituted and with 1 to 20 amino acid residues being added to the carboxyl terminal and / or amino terminal of sequence 4;
4) Drosophila melanogas Glyrichin: protein having the amino acid residue sequence of the sequence 5 in the Sequence List or protein with antibacterial activities having sequence 5 in the Sequence List with 1 to 20 amino acid residues of it being deleted, inserted and / or substituted and with 1 to 20 amino acid residues being added to the carboxyl terminal and / or amino terminal of sequence 5;
5) Caenorhabditis elegans Glyrichin: protein having the amino acid residue sequence of the sequence 6 in the Sequence List or protein with antibacterial activities having sequence 6 in the Sequence List with 1 to 20 amino acid residues of it being deleted, inserted and / or substituted and with 1 to 20 amino acid residues being added to the carboxyl terminal and / or amino terminal of sequence 6;
6) Caenorhabditis elegans Glyrichin: protein having the amino acid residue sequence of the sequence 7 in the Sequence List or protein with antibacterial activities having sequence 7 in the Sequence List with 1 to 20 amino acid residues of it being deleted, inserted and / or substituted and with 1 to 20 amino acid residues being added to the carboxyl terminal and / or amino terminal of sequence 7;
7) Schizosaccharomyces pombe Glyrichin: protein having the amino acid residue sequence of the sequence 8 in the Sequence List or protein with antibacterial activities having sequence 8 in the Sequence List with 1 to 20 amino acid residues of it being deleted, inserted and / or substituted and with 1 to 20 amino acid residues being added to the carboxyl terminal and / or amino terminal of sequence 8;
8) Sacchromyces cerevisiae Glyrichin: protein having the amino acid residue sequence of the sequence 9 in the Sequence List or protein with antibacterial activities having sequence 9 in the Sequence List with 1 to 20 amino acid residues of it being deleted, inserted and / or substituted and with 1 to 20 amino acid residues being added to the carboxyl terminal and / or amino terminal of sequence 9;
9) Arabiopsis thaliana Glyrichin: protein having the amino acid residue sequence of the sequence 10 in the Sequence List or protein with antibacterial activities having sequence 10 in the Sequence List with 1 to 20 amino acid residues of it being deleted, inserted and / or substituted and with 1 to 20 amino acid residues being added to the carboxyl terminal and / or amino terminal of sequence 10;
10) Plasmodium falciparum 3D7 Glyrichin: protein having the amino acid residue sequence of the sequence 11 in the Sequence List or protein with antibacterial activities having sequence 11 in the Sequence List with 1 to 20 amino acid residues of it being deleted, inserted and / or substituted and with 1 to 20 amino acid residues being added to the carboxyl terminal and / or amino terminal of sequence 11;
11) Plasmodium yoelii yoelii Glyrichin: protein having the amino acid residue sequence of the sequence 12 in the Sequence List or protein with antibacterial activities having sequence 12 in the Sequence List with 1 to 20 amino acid residues of it being deleted, inserted and / or substituted and with 1 to 20 amino acid residues being added to the carboxyl terminal and / or amino terminal of sequence 12;
12)Magnaporthe grisea Glyrichin: protein having the amino acid residue sequence of the sequence 13 in the Sequence List or protein with antibacterial activities having sequence 13 in the Sequence List with 1 to 20 amino acid residues of it being deleted, inserted and / or substituted and with 1 to 20 amino acid residues being added to the carboxyl terminal and / or amino terminal of sequence 13;
13) Neurospora crassa Glyrichin: protein having the amino acid residue sequence of the sequence 14 in the Sequence List or protein with antibacterial activities having sequence 14 in the Sequence List with 1 to 20 amino acid residues of it being deleted, inserted and / or substituted and with 1 to 20 amino acid residues being added to the carboxyl terminal and / or amino terminal of sequence 14.

2. The Glyrichin of Claim 1, **characterizing in that** said Glyrichin are hGlyrichin and mGlyrichin, which are proteins having amino acid residues in Sequence 1 in the Sequence List or protein with antibacterial activities having sequence 1 in the Sequence List with 1 to 20 amino acid residues of it being deleted, inserted and / or substituted and with 1 to 20 amino acid residues being added to the carboxyl terminal and / or amino terminal of sequence 1.

3. The Glyrichin of Claim 1 or 2, **characterizing in that** the number of the amino acid residues deleted, inserted and/or substituted and added at the carboxyl terminal or amino terminal is 1 to 10.

4. The Glyrichin of Claim 3, **characterizing in that** the number of the amino acid residues deleted, inserted and/or substituted and added at the carboxyl terminal or amino terminal is 1 to 5.

5. The Glyrichin of Claim 4, **characterizing in that** the number of the amino acid residues deleted, inserted and/or substituted and added at the carboxyl terminal or amino terminal is 1 to 3.

6. A coding gene of the Glyrichin according to any one of Claims 1 to 5.

7. The gene of Claim 6, **characterizing in that** said Glyrichin is hGlyrichin and its coding gene has DNA sequence in the SEQID No:2 of Sequence List or has >90% homology with the DNA sequence in the SEQID No:2 of the Sequence List, and the DNA sequence of amino acid residue sequence in the coding Sequence List 1 or nucleotide sequences which can hybridize with DNA sequence in the Sequence 2 of Sequence List under high strict condition.

8. A expression vector containing genes according to Claim 6 or 7.

9. A cell line containing genes according to Claim 6 or 7.

10. An engineering bacteria containing genes according to Claim 6 or 7.

11. An antibacterial use of the Glyrichin according to any one of Claims 1 to 5 as well as the coding gene thereof.

12. The use of Claim 11, **characterizing in that** the Glyrichin and the coding gene thereof are applied in preparing drugs for prevention and/or treatment of bacterial infectious disease of human or livestock.

13. The use of Claim 11, **characterizing in that** the Glyrichin and the coding gene thereof are applied in preparing drugs for prevention and/or treatment of potentially bacterial infectious disease of different kinds of creatures.

14. The use of Claim 11, **characterizing in that** the Glyrichin and the coding gene thereof are applied in producing transgenic creatures that can defend against diseases and pests.

15. The use of Claim 11, **characterizing in that** the Glyrichin and the coding gene thereof are applied in preparing the derivatives, or antagonists as well as its ligands and antibodies of Glyrichin.
